(19) Europäisches Patentamt
European Patent Office
Office européen des brevets

(11) Publication number: **0 182 407**
**B1**

(12) **EUROPEAN PATENT SPECIFICATION**

(45) Date of publication of patent specification: **16.05.90**

(51) Int. Cl.[5]: **C 07 C 251/56,** C 07 C 251/60, C 07 C 323/22

(21) Application number: **85201699.7**

(22) Date of filing: **15.10.85**

(54) **Alpha-aminooxy C4-alkanoic acids and esters.**

(30) Priority: **18.10.84 US 662117**

(43) Date of publication of application:
**28.05.86 Bulletin 86/22**

(45) Publication of the grant of the patent:
**16.05.90 Bulletin 90/20**

(84) Designated Contracting States:
**AT BE CH DE FR GB IT LI LU NL SE**

(56) References cited:
**DE-A-2 455 353**
**DE-A-2 455 432**
**DE-A-2 812 366**
**DE-B-1 960 910**
**FR-A-2 511 364**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Sanborn, James Russell**
**5612 Chenault Drive**
**Modesto California 95356 (US)**
Inventor: **Tieman, Charles Henry**
**2209 Fremont Street**
**Modesto California 95350 (US)**

(74) Representative: **Bennett, David Arthur Horder et al**
**4, York Road**
**London SE1 7NA (GB)**

**EP 0 182 407 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to new alpha-aminooxy $C_4$—$C_6$ alkanoic acids and esters and certain derivatives thereof, their use as herbicides and plant growth regulators and to herbicidal plant growth regulator formulations containing these new compounds.

The present invention is directed to new alpha-aminooxy $C_4$—$C_6$ alkanoic acid and esters of the Formula I

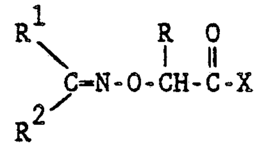

(I)

wherein X is OH or $OR^3$ in which $R^3$ is an alkyl or alkoxyalkyl group containing from 1 to 10 carbon atoms; R is an alkyl group containing from 2 to 4 carbon atoms; $R^1$ is a hydrogen atom or an alkyl group containing from 1 to 6 carbon atoms optionally substituted by one or more halogen atoms; and $R^2$ is an alkyl or an alkenyl group containing up to 6 carbon atoms, a phenyl group optionally substituted by one or more substituents selected from a halogen atom, a nitro group, an alkyl, alkylthio or alkoxy group containing from 1 to 4 carbon atoms each optionally substituted by one or more halogen atoms, or by a methylenedioxy group, or $R^1$ and $R^2$ together with the carbon atom to which they are attached form a cyclic hydrocarbyl group containing from 3 to 12 carbon atoms. The compounds are useful as herbicides and plant growth regulators.

Examples of individual species within the scope of the invention include:

Butanoic acid, 2-(((1-(4-bromophenyl)ethylidene)amino)oxy)-, methyl ester,

Butanoic acid, 3-methyl-2(((1-(3-trifluoromethyl)phenyl)ethylidene)amino)oxy)-, methyl ester,

Butanoic acid, 2-(((1-(3-(trifluoromethoxy)phenyl)ethylidene)amino)oxy)- tert-butyl ester,

Butanoic acid, 2-(((1-(4-ethylthio)phenyl)ethylidene)amino)oxy)-.

The halogen atoms to which reference is made in the definitions following Formula I include chlorine, fluorine and bromine.

In the compounds of the invention, R is preferably an ethyl or isopropyl group, $R^1$ is preferably a hydrogen atom or a methyl, ethyl or trifluoromethyl group and $R^2$ is preferably a methyl group, a 2-methylpropenyl group, a phenyl group substituted by one or two groups selected from chlorine, fluorine, nitro, methyl or trifluoromethyl, or by methylenedioxy, for example 4-chlorophenyl, 2-chlorophenyl, 2,4-dichlorophenyl, 2-methylpropenyl, 3,4-methylenedioxyphenyl, 3-(trifluoromethyl)phenyl, or is a tetralinyl group.

When $R_1$ is a hydrogen atom, $R_2$ is preferably a 2,4-dichlorophenyl or 3-nitrophenyl group; and when $R_1$ is a methyl group $R_2$ is preferably a 3-trifluoromethylphenyl group.

This invention is directed to novel chemical compounds and the use of certain active compounds in regulating the natural growth or development of plants. In particular, this invention relates to the chemical treatment of plants to alter their natural growth or development for the purpose of enhancing various agricultural or horticultural features of the plants, and also to the control of undesirable vegetation, including killing.

This invention further relates to a method of controlling plant growth by regulating the natural growth or development of plants comprising applying to said plants an effective, plant-regulating, non-lethal amount of the above compounds, as well as controlling undesirable vegetation comprising applying a herbicidally effective amount of the compounds to such vegetation.

As employed herein, the term "natural growth or development" designates the normal life cycle of a plant in accordance with its genetics and environment, in the absence of artificial external influences.

The term "regulating" is used herein to denote the bringing about through chemical means of any temporary or permanent modification or variation from the normal life cycle short of killing the plant.

With properly controlled application, a growth regulating effect can be achieved without herbicidal results. The amount which constitutes an effective amount varies not only with the particular material selected for treatment, but also with the regulatory effect to be achieved, the species of plant being treated and its stage of development, and whether a permanent or transient effect is sought.

Growth regulation may arise from the effect of the compound on either the physiological processes or the morphology of the plant, or from both in combination or in sequence. Morphological changes are generally noticible by observable changes in the size, shape, colour or texture of the treated plant or any of its parts, as well as in the quantity of fruit or flowers produced. Changes in physiological processes on the other hand, occur within the treated plant and are usually hidden from the eye of an observer. Changes of this type most often occur in the production, location, storage or use of chemicals naturally occurring in the plant, such as hormones. Physiological changes may be visually detectable when followed by a change in morphology. In addition, numerous analytical procedures for determining the nature and magnitude of changes in the various physiological processes are known to those skilled in the art.

The active compounds of the instant invention serve to regulate the natural growth or development of treated plants in a number of diverse ways, and it is to be understood that each compound may not

produce identical regulatory effects on each plant species or at every rate of application. As stated above, responses will vary in accordance with the compound, the rate, the plant, etc.

The term "herbicidally effective amount" designates any amount of the compounds disclosed herein which will kill a plant or any portion thereof.

By "plants" is meant germinant seeds, emerging seedlings, and established vegetation, including roots and above-ground portions. Effects include killing, defoliation, desiccation, stunting, leaf burn, and dwarfing. Herbicidal effects are generally achieved at higher application rates than growth regulating effects.

The compounds of the invention can exhibit geometrical and optical isomerism. The present invention includes all the biologically active compounds of Formula I of the invention and thus includes racemates and various geometric and/or optically-active isomers or mixtures enriched in such isomer forms which may be directly synthesized, resolved or mixed together. The various geometrical and optical isomers of the compounds of Formula I may have different biological activity.

The alpha-aminooxy $C_4$—$C_6$ alkanoic isovaleric acids and esters of Formula I may be prepared by forming the alkali metal salt of an oxime with alkali metal hydroxide in an inert solvent (e.g. toluene) in the presence of a catalyst (including onium catalysts) with azeotropic distillation of the water formed. After the water is removed, a 2-bromoalkanoic acid alkyl ester is added and the reaction mixture is refluxed, often for 24—48 hours. The reaction mixture was filtered to remove the salts prior to distillation in a Kugelrohr apparatus. The carboxylic acids were prepared via alkaline hydrolysis in a ternary mixture of ethanol-tetrahydrofuran and water.

The oximes utilized in making the acids and alkyl esters of this invention were prepared from the corresponding carbonyl compound and hydroxylamine hydrochloride via well known methods, e.g., Vogel, A. I., Practical Organic Chemistry, Wiley and Sons, 1966, p. 343.

The invention is illustrated by the following examples, which are presented for the purpose of illustration. The identity of the products, including intermediates, was confirmed by elemental, infrared and nuclear magnetic resonance spectral (NMR) analysis as necessary.

Example 1

Butanoic acid, 3-methyl-2(((1-3-(trifluoromethyl)phenyl)ethylidine)amino)oxy-, ethyl ester.

In a 500 ml round bottom flask fitted with a magnetic stirrer, Dean-Stark trap and nitrogen purge were placed 2.0 g sodium hydroxide in 15 ml of water. To this was added 10.16 g of 3-(trifluoromethyl)-acetophenone oxime in 15 ml of toluene and a catalytic amount of tetrabutylammonium hydrogen sulfate and the mixture was heated to reflux overnight. After cooling, 9.75 g of ethyl 2-bromo-3-methyl butyrate was added all at once and the mixture was refluxed for three days. After cooling, the reaction mixture was filtered and the product was distilled in a Kugelrohr apparatus to yield 7.33 g of the desired product; b.p. 90°C (6.67 Pa—0.05 mm).

Example 2

Butanoic acid, 3-methyl-2(((1-(3-(trifluoromethyl)phenyl)ethylidine)amino)oxy-.

In a 300 ml round bottom flask equipped with a magnetic stirrer and reflux condenser were placed 15 ml water, 15 ml of tetrahydrofuran, 15 ml of ethanol, 0.6 g of sodium hydroxide and 5.0 g of the above ester of Example 1. This mixture was refluxed for 36 hours. The reaction mixture was evaporated to dryness and the residue was taken up in 10% sodium hydroxide. The basic solution was then extracted with methylene chloride to remove neutral products. Acidification of the basic solution and extraction of the aqueous solution with methylene chloride yielded, after drying of the methylene chloride with magnesium sulfate and evaporating the solvent, 4.21 g of the desired product; m.p. 49—51°C.

Examples 3—64

Following the procedures similar to those described in Examples 1 and 2 above, additional alpha-aminooxy $C_4$—$C_6$ alkanoic acids and esters of Formula I were prepared as set forth in Table 1 below.

Table 1. ALPHA-AMINOOXY $C_4$-$C_6$ ALKANOIC ACIDS AND ESTERS OF FORMULA I

| Embodiment | R | $R^1$ | $R^2$ | X |
|---|---|---|---|---|
| 3 | i-$C_3H_7$ | $CH_3$ | phenyl | OH |
| 4 | i-$C_3H_7$ | H | " | OH |
| 5 | i-$C_3H_7$ | $-CH—C(CH_3)_2-CH_2-CH(CH_3)-CH-$ | | OH |
| 6 | i-$C_3H_7$ | $CH_3$ | $-C=C(CH_3)_2$ | OH |
| 7 | i-$C_3H_7$ | $C_2H_5$ | phenyl | OH |
| 8 | i-$C_3H_7$ | 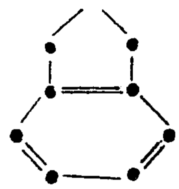 | | OH |
| 9 | i-$C_3H_7$ | $CH_3$ | 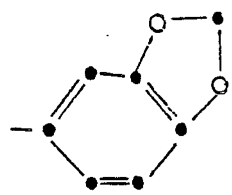 | OH |
| 10 | i-$C_3H_7$ | $CH_3$ | $CH_3$ | OH |
| 11 | i-$C_3H_7$ | H | 4-Cl phenyl | $OC_2H_5$ |
| 12 | i-$C_3H_7$ | H | phenyl | $OC_2H_5$ |
| 13 | i-$C_3H_7$ | $CH_3$ | 4-Cl phenyl | OH |
| 14 | i-$C_3H_7$ | H | " | OH |
| 15 | i-$C_3H_7$ | $CH_3$ | 2-Cl phenyl | $OC_2H_5$ |
| 16 | $C_2H_5$ | H | phenyl | $OC_2H_5$ |
| 17 | $C_2H_5$ | H | phenyl | OH |
| 18 | i-$C_3H_7$ | $CH_3$ | 2-Cl phenyl | OH |
| 19 | i-$C_3H_7$ | 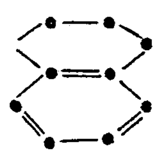 | | $OC_2H_5$ |
| 20 | i-$C_3H_7$ | 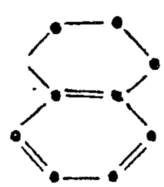 | | OH |

| Embodiment | R | $R^1$ | $R^2$ | X |
|---|---|---|---|---|
| 21 | $i-C_3H_7$ | $CH_3$ | 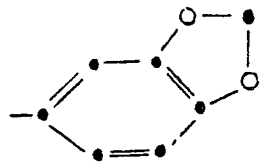 | $OC_2H_5$ |
| 22 | $i-C_3H_7$ | H | phenyl | $OCH_3$ |
| 23 | $i-C_3H_7$ | H | 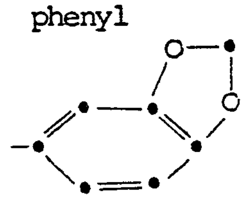 | $OC_2H_5$ |
| 24 | $i-C_3H_7$ | H | 4-$CH_3$S-phenyl | $OC_2H_5$ |
| 25 | $i-C_3H_7$ | H | -2,4-$Cl_2$phenyl | $OC_2H_5$ |
| 26 | $i-C_3H_7$ | H | 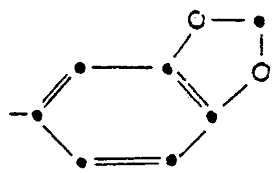 | OH |
| 27 | $i-C_3H_7$ | H | 2,4-$Cl_2$phenyl | OH |
| 28 | $i-C_3H_7$ | H | 4-thiomethylphenyl | OH |
| 29 | $i-C_3H_7$ | H | 4-methoxy phenyl | $OC_2H_5$ |
| 30 | $i-C_3H_7$ | H | 4-$CH_3$O phenyl | OH |
| 31 | $i-C_3H_7$ | H | 2-Cl phenyl | $OC_2H_5$ |
| 32 | $i-C_3H_7$ | H | 2-Cl phenyl | OH |
| 33 | $i-C_3H_7$ | $CH_3$ | 4-$CH_3$ phenyl | $OC_2H_5$ |
| 34 | $i-C_3H_7$ | $CH_3$ | 4-$CH_3$ phenyl | OH |
| 35 | $i-C_3H_7$ | H | 4-F phenyl | $OCH_2H_5$ |
| 36 | $C_2H_5$ | $CH_3$ | 4-Cl phenyl | $OC_2H_5$ |
| 37 | $C_2H_5$ | H | 4-Cl phenyl | $OC_2H_5$ |
| 38 | $i-C_3H_7$ | H | 4-F phenyl | OH |
| 39 | $C_2H_5$ | $CH_3$ | 4-Cl phenyl | OH |
| 40 | $C_2H_5$ | H | 4-Cl phenyl | OH |
| 41 | $i-C_3H_7$ | H | 4-$CH_3$ phenyl | $OC_2H_5$ |
| 42 | $i-C_3H_7$ | H | 4-$CH_3$ phenyl | OH |

| Embodiment | R | $R^1$ | $R^2$ | X |
|---|---|---|---|---|
| 43 | i-$C_3H_7$ | -CH—C($CH_3$)-$CH_2$-CH($CH_3$)-CH- | | $OCH_2OCH_3$ |
| 44 | i-$C_3H_7$ | H | 4-$CH_3$ phenyl | $OC_2H_5$ |
| 45 | i-$C_3H_7$ | $CH_3$ | phenyl | $OC_2H_5$ |
| 46 | i-$C_3H_7$ | H | 3-$CF_3$ phenyl | $OC_2H_5$ |
| 47 | i-$C_3H_7$ | H | 3-$CF_3$ phenyl | OH |
| 48 | i-$C_3H_7$ | H | 3-$CF_3$ phenyl | OH |
| 49 | i-$C_3H_7$ | H | 3,4-$Cl_2$ phenyl | $OC_2H_5$ |
| 50 | i-$C_3H_7$ | $CH_3$ | 3,4-$Cl_2$ phenyl | OH |
| 51 | i-$C_3H_7$ | $CH_3$ | 3-$CF_3$ phenyl | OH |
| 52 | i-$C_3H_7$ | H | 4-$NO_2$ phenyl | $OC_2H_5$ |
| 53 | i-$C_3H_7$ | H | 3-$NO_2$ phenyl | OH |
| 54 | i-$C_3H_7$ | H | 4-$NO_2$ phenyl | OH |
| 55 | i-$C_3H_7$ | $CF_3$ | phenyl | $OC_2H_5$ |
| 56 | i-$C_3H_7$ | $CF_3$ | phenyl | OH |
| 57 | i-$C_3H_7$ | H | 4-methylcyclohexenyl | $OC_2H_5$ |
| 58 | $C_2H_5$ | H | 3-$NO_2$ phenyl | OH |
| 59 | n-$C_4H_9$ | H | 3-$NO_2$ phenyl | $OC_2H_5$ |
| 60 | n-$C_3H_7$ | H | 3-$NO_2$ phenyl | $OC_2H_5$ |
| 61 | i-$C_3H_7$ | $CH_3$ | 3-$NO_2$ phenyl | $OC_2H_5$ |
| 62 | n-$C_4H_9$ | H | 3-$NO_2$ phenyl | OH |
| 63 | $C_2H_5$ | H | 3-$NO_2$ phenyl | $OC_2H_5$ |
| 64 | i-$C_3H_7$ | $CH_3$ | $CH_3$ | $OC_2H_5$ |

For application, the compound of Formula I ordinarily is applied most effectively by formulating it with a suitable inert carrier or surface-active agent, or both. The invention, therefore, also includes compositions suitable for combatting unwanted plants, such compositions comprising an inert carrier or surface-active agent, or both, and as active ingredient at least one compound of Formula I.

The term "carrier" as used herein means an inert solid or liquid material, which may be inorganic or organic and of synthetic or natural origin, with which the active compound is mixed or formulated to facilitate its application to the plant, seed, soil or other object to be treated, or its storage, transport and/or handling. Any of the materials customarily employed in formulating pesticides, herbicides, or fungicides, are suitable.

Suitable solid carriers are natural and synethic clays and silicates, for example, talcs; magnesium aluminum silicates, for example attapulgites and vermiculites; aluminum silicates, for example kaolinites, montmorillonites and micas; calcium carbonate; calcium sulfate; synthetic hydrated silicon oxides and synthetic calcium or aluminum silicates; elements such as, for example, carbon and sulfur; natural and synthetic resins such as, for example, coumarone resins, polyvinyl chloride and styrene polymers and copolymers; bitumen; waxes such as, for example, beeswax, paraffin wax, and chlorinated mineral waxes; solid fertilizers, for example, superphosphates; and ground, naturally-occurring, fibrous materials, such as ground corncobs.

Examples of suitable liquid carriers are water, alcohols such as isopropyl alcohol and glycols; ketones such as acetone, methyl ethyl ketone, methyl isobutyl ketone and cyclohexanone; ethers such as cellosolves; aromatic hydrocarbons such as benzene, toluene and xylene; petroleum fractions such as kerosene, light mineral oils; chlorinated hydrocarbons such as carbon tetrachloride, perchloroethylene and trichloromethane. Also suitable are liquefied, normally vaporous and gaseous compounds. Mixtures of different liquids are often suitable.

The surface-active agent may be an emulsifying agent or a dispersing agent or a wetting agent; it may be nonionic or ionic. Any of the surface-active agents usually applied in formulating herbicides or insecticides may be used. Examples of suitable surface-active agents are the sodium and calcium salts of polyacrylic acids and lignin sulfonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols or alkyl phenols, for example, p-octylphenol or p-octylcresol, with ethylene oxide and/or propylene oxide; sulfates or sulfonates of these condensation products, alkali or alkaline earth metal salts, preferably sodium salts, of sulfuric or sulfonic acid esters containing at least 10 carbon atoms in the molecule, for example, sodium lauryl sulfate, sodium secondary alkyl sulfates, sodium salts of sulfonated castor oil, and sodium alkylaryl sulfonates such as sodium dodecylbenzene sulfonate; and polymers of ethylene oxide and copolymers of ethylene oxide and propylene oxides.

The compositions of the invention may be prepared as wettable powders, dusts, granules, solutions, emulsifiable concentrates, emulsions, suspension concentrates and aerosols. Wettable powders are usually compounded to contain 25 to 75% by weight of active compound and usually contain, in addition to the solid carrier, 3—10% by weight of a dispersing agent, 2—15% of a surface-active agent and, where necessary, 0—10% by weight of stabilizer(s) and/or other additives such as penetrants or stickers. Dusts are usually formulated as a dust concentrate having a similar composition to that of a wettable powder but without a dispersant or surface-active agent, and are diluted in the field with further solid carrier to give a composition usually containing 0—5—10% by weight of the active compound. Granules are usually prepared to have a size between 10 and 1000 BS mesh (1.676—0.152 mm), and may be manufactured by agglomeration or impregnation techniques. Generally, granules will contain 0.5—25% by weight of the active compound, 0—1% by weight of additives such as stabilizers, slow release modifiers and binding agents. Emulsifiable concentrates usually contain, in addition to the solvent and, when necessary, cosolvent, 10—50% weight per volume of the active compound, 2—20% weight per volume emulsifiers and 0—20% weight per volume of appropriate additives such as stabilizers, penetrants and corrosion inhibitors. Suspension concentrates are compounded so as to obtain a stable, non-sedimenting, flowable product and usually contain 10—75% weight of the active compound, 0.5—5% weight of dispersing agents, 1—5% of surface-active agent, 0.1—10% weight of suspending agents, such as defoamers, corrosion inhibitors, stabilizers, penetrants and stickers, and as carrier, water or an organic liquid in which the active compound is substantially insoluble; certain organic solids or inorganic salts may be dissolved in the carrier to assist in preventing sedimentation or as antifreeze agents for water.

Of particular interest in current practice are water-dispersible granular formulations. These are in the form of dry, hard granules that are essentially dust-free, and are resistant to attrition on handling, thus minimizing the formation of dust. On contact with water, the granules readily disintegrate to form stable suspensions of the particles of active material. Such formulations contain 90% or (up to 95%) more by weight of finely divided active material, 3—7% by weight of a blend of surfactants, which act as wetting, dispersing, suspending and binding agents, and may contain up to 3% by weight of a finely divided carrier, which acts as a resuspending agent.

Aqueous dispersions and emulsions, for example, compositions obtained by diluting a wettable powder or a concentrate according to the invention with water, also lie within the scope of the present invention. The said emulsions may be of the water-in-oil or of the oil-in-water type, and may have thick, mayonnaise-like consistency.

It is evident from the foregoing that this invention contemplates compositions containing as little as about 0.5% by weight to as much as about 95% by weight of a compound of Formula I as the active ingredient.

The compositions of the invention may also contain other ingredients, for example, other compounds possessing pesticidal, especially insecticidal, acaricidal, herbicidal or fungicidal properties, as are appropriate to the intended purpose.

As mentioned above, the compounds of the Invention have been found useful for influencing or regulating plant growth and controlling (the growth of) unwanted plants, being particularly active with respect to one or more grassy or broadleafed weeds or plants. For example, the compounds can change plant morphology; depress the growth of plants; inhibit germination; or totally or selectively kill plants depending on the compounds and the amount used. As herbicides, they appear to be more effective when applied preemergence or pre-plant incorporated (applied to the soil before the seeds have sprouted) than when applied postemergence (applied to the foliage).

Protection of a locus or area from undesirable plants is effected by applying a compound of Formula I, ordinarily in a composition of one of the aforementioned types, to soil in which the seeds of the unwanted plants are present, or to the foliage of the unwanted plants. The active compound, of course, is applied in an amount sufficient to exert the desired action.

The amount of the compound of the invention to be used in combatting undesired plants will naturally depend on the condition of the plants, the degree of activity desired, the formulation used, the mode of application, the climate, the season of the year, and other variables. Recommendations as to precise amounts are, therefore, not possible. In general, however, application to the locus to be protected of from

0.1 to 10.0 kg per hectare of the compound of Formula I will be satisfactory.

Examples of Activity with Respect to Plants

In the following examples, the species of plants that were tested were:

Barnyardgrass (watergrass) — *Echinochloa crus-galli*
Large crabgrass — *Digitaria sanguinalis*
Downy brome — *Bromus tectorum*
Yellow foxtail — *Setaria lutescens*
Redroot pigweed — *Amaranthus retroflexus*
Sicklepod — *Cassia obtusifolia*
Velvet leaf — *Abutilon theophrasti*
Garden cress — *Lepidium sativum*
Johnson grass — *Sorghum halepense*
Morningglory — *Ipomoea purpurea* L. (Roth)

Test Procedures

The preemergence (soil) herbicidal activity of compounds of Formula I was evaluated by planting seeds of barnyardgrass, garden cress, downy brome, velvetleaf, yellow foxtail, and either sicklepod or morningglory in test tubes, nominally measuring 25 × 200 millimeters, filled about three-quarters full of untreated soil, in each case covered on top with about 2.5 cubic centimeters of soil treated with a certain amount of the test compound. The treated soil applied to the tubes containing the barnyardgrass and cress seeds contained one milligram of the test compound per tube, and contained 0.1 milligram of the test compound per each tube containing the seeds of the other plants. The dosages were approximately 20 and 2.0 kg of test compound per hectare, respectively. The seeds were planted on top of the treated soil and covered with about 1.5 cubic centimeters of untreated soil. The planted soil was held under controlled conditions of temperature, moisture, and light for 9 to 10 days. The amounts of germination and growth in each tube were evaluated on a 0 to 9 scale, and numeric ratings having the following meanings:

| Rating | Meaning |
|---|---|
| 9 | No living tissue |
| 8 | Plant severely damaged and expected to die |
| 7 | Plant badly damaged, but expected to live |
| 6 | Moderate damage, but complete recovery expected |
| 5 | Intermediate damage (probably unacceptable for crop plants) |
| 3—4 | Observable damage |
| 1—2 | Plant slightly affected, possibly by the chemical, possibly due to biological variability |
| 0 | No visible effect |

The postemergence (foliar) herbicidal activity of compounds of Formula I was evaluated by spraying 10-day-old large crabgrass plants, 13-day-old pigweed plants, 6-day-old Johnsongrass plants, 9-day-old velvetleaf plants, 9-day-old yellow foxtail plants and either 9-day-old sicklepod plants or 5-day-old morningglory plants to runoff with a liquid formulation of the test compound. The crabgrass and pigweed plants were sprayed with 2.4 milliliters of a 0.25% solution (about ten kg of the test compound per hectare), and other plants were sprayed with 2.4 milliliters of a 0.025% solution (about one kg of the test compound per hectare). The sprayed plants were held under controlled conditions of temperature, moisture and light for 7 to 8 days, when the effect of the test compound was evaluated visually, the results being rated on the 0 to 9 scale described above.

Illustrative results of the preemergence and postemergence herbicidal activity tests are set forth below.

In Preemergence tests,

(a) on barnyard grass, the following compounds were rated 7 or better:

Compounds of Embodiments 3, 10, 14, 16, 17, 18, 20, 26, 39, 40, 47, 48, 50, 51, 55

(b) on garden cress, the following compounds were rated 7 or better:

Compounds of Embodiments 3, 4, 5, 7, 8, 9, 10, 13, 14, 16, 17, 18, 20, 23, 26, 30, 32, 34, 36, 37, 38, 39, 40, 42, 47, 48, 50, 51, 52, 53, 54, 56, 57, 58, 60, 62, 63

In Postemergence tests,

(a) on large crabgrass, the following compounds were rated 7 or better:

Compounds of Embodiments 10, 50, 51

(b) on pigweed, the following compounds were rated 7 or better:

Compounds of Embodiments 7, 9, 13, 14, 18, 20, 21, 23, 30, 32, 36, 37, 38, 39, 40, 47, 48, 49, 50, 51, 54, 56, 58, 60, 63

In addition, almost all of the compounds of the invention, demonstrated one or more effects on plants, such as depressing growth, dwarfing, inhibiting germination, chlorosis, necroses, etc. against one or more of the test plant species.

**Claims for the Contracting States: BE CH DE FR GB IT LI LU NL SE**

1. A compound of formula I

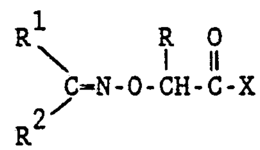

(I)

wherein X is OH or $OR^3$ in which $R^3$ is an alkyl or alkoxyalkyl group containing from 1 to 10 carbon atoms; R is an alkyl group containing from 2 to 4 carbon atoms; $R^1$ is a hydrogen atom or an alkyl group containing from 1 to 6 carbon atoms optionally substituted by one or more halogen atoms; and $R^2$ is an alkyl or an alkenyl group containing up to 6 carbon atoms, a phenyl group optionally substituted by one or more substituents selected from a halogen atom, a nitro group, an alkyl, alkylthio or alkoxy group containing from 1 to 4 carbon atoms each optionally substituted by one or more halogen atoms, or by a methylenedioxy group, or $R^1$ and $R^2$ together with the carbon atom to which they are attached form a cyclic hydrocarbyl group containing from 3 to 12 carbon atoms.

2. A compound as claimed in claim 1 wherein R is an ethyl or ispropyl group; $R^1$ is a hydrogen atom or methyl, ethyl or trifluoromethyl group; and $R^2$ is a methyl group, a 2-methylpropenyl group, a phenyl group substituted by one or two groups selected from chlorine, fluorine, nitro, methyl or trifluoromethyl or by methylenedioxy, or is a tetralinyl group.

3. A compound as claimed in claim 2 wherein $R^1$ is a hydrogen atom and $R^2$ is 2,4-dichlorophenyl, or 3-nitrophenyl.

4. A compound as claimed in claim 2 wherein $R^1$ is a methyl group and $R^2$ is a 3-trifluoromethylphenyl group.

5. A plant growth regulating composition comprising a plant growth regulating amount of an active compound according to claim 1, together with at least one carrier or surface-active agent.

6. A method of controlling plant growth at a locus comprises applying to the locus or the plants a controlling amount of an active compound according to claim 1, or a composition as claimed in claim 5.

**Claims for the Contracting State: AT**

1. A plant growth regulating composition comprising a plant growth regulating amount of a compound of formula I

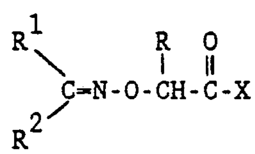

(I)

wherein X is OH or $OR^3$ in which $R^3$ is an alkyl or alkoxyalkyl group containing from 1 to 10 carbon atoms; R is an alkyl group containing from 2 to 4 carbon atoms; $R^1$ is a hydrogen atom or an alkyl group containing from 1 to 6 carbon atoms optionally substituted by one or more halogen atoms; and $R^2$ is an alkyl or an alkenyl group containing up to 6 carbon atoms, a phenyl group optionally substituted by one or more substituents selected from a halogen atom, a nitro group, an alkyl, alkylthio or alkoxy group containing from 1 to 4 carbon atoms each optionally substituted by one or more halogen atoms, or by a methylenedioxy group, or $R^1$ and $R^2$ together with the carbon atom to which they are attached form a cyclic hydrocarbyl group containing from 3 to 12 carbon atoms, together with at least one carrier or surface-active agent.

2. A composition as claimed in claim 1, wherein, in formula I, R is an ethyl or ispropyl group; $R^1$ is a hydrogen atom or methyl, ethyl or trifluoromethyl group; and $R^2$ is a methyl group, a 2-methylpropenyl group, a phenyl group substituted by one or two groups selected from chlorine, fluorine, nitro, methyl or trifluoromethyl or by methylenedioxy, or is a tetralinyl group.

3. A composition as claimed in claim 2, wherein $R^1$ is a hydrogen atom and $R^2$ is 2,4-dichlorophenyl, or 3-nitrophenyl.

4. A composition as claimed in claim 2, wherein $R^1$ is a methyl group and $R^2$ is a 3-trifluoromethylphenyl group.

5. A method of controlling plant growth at a locus comprises applying to the locus or the plants a controlling amount of a compound as defined in claim 1, or a composition as claimed in claim 1.

**Patentansprüche für die Vertragsstaatten: BE CH DE FR GB IT LI LU NL SE**

1. Verbindung der Formel I

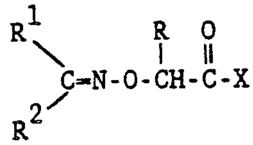

(I)

worin X für OH oder $OR^3$ steht, $R^3$ eine Alkyl- oder Alkoxyalkylgruppe mit 1 bis 10 Kohlenstoffatomen ist; R eine Alkylgruppe mit 2 bis 4 Kohlenstoffatomen darstellt; $R^1$ ein Wasserstoffatom oder eine Alkylgruppe mit 1 bis 6 Kohlenstoffatomen darstellt, welche, wahlweise durch eine oder mehrer Halogenatome substituiert ist; und $R^2$ eine Alkyl- oder eine Alkenylgruppe mit bis zu 6 Kohlenstoffatomen, eine Phenylgruppe ist, welche Phenylgruppe wahlweise durch einen oder mehrere Substituenten, ausgewählte unter einem Halogenatom, einer Nitrogruppe, einer Alkyl-, Alkylthio- oder Alkoxygruppe mit 1 bis 4 Kohlenstoffatomen, welche jeweils wahlweise durch ein oder mehrere Halogenatome substituiert sind, oder durch eine Methylendioxygruppe substituiert ist, oder $R^1$ und $R^2$ zusammen mit dem Kohlenstoffatom, an welches sie begunden sind, eine cyklische Kohlenwasserstoffgruppe mit 3 bis 12 Kohlenstoffatomen bilden.

2. Verbindung nach Anspruch 1, worin R eine Ethyl- oder Isopropylgruppe ist; $R^1$ ein Wasserstoffatom oder eine Methyl-, Ethyl- oder Trifluoromethylgruppe darstellt; und $R^2$ eine Methylgruppe, eine 2-Methylpropenylgruppe, eine Phenylgruppe darstellt, welche durch eine oder zwei Gruppen, ausgewählt unter Chlor, Fluor, Nitro, Methyl oder Trifluoromethyl, oder durch Methylendioxy substituiert ist, oder eine Tetralinylgruppe ist.

3. Verbindung nach Anspruch 2, worin $R^1$ ein Wasserstoffatom ist und $R^2$ 2,4-Dichlorphenyl oder 3-Nitrophenyl darstellt.

4. Verbindung nach Anspruch 2, worin $R^1$ eine Methylgruppe ist und $R^2$ eine 3-Trifluormethylphenylenegruppe ist.

5. Pflanzenwuchsregulierende Zusammensetzung, welche eine pflanzenwuchsregulierende Menge einer wirksamen Verbindung nach Anspruch 1, gemeinsam mit mindestens einem Träger oder einem oberflächenaktiven Mittel umfaßt.

6. Verfahrens zur Bekämpfung von Pflanzenwuchs an einem Ort, welches das Aufbringen einer bekämpfenden Menge einer wirksamen Verbindung nach Anspruch 1 oder eine Zusammensetzung nach Anspruch 5 auf den Ort oder die Pflanzen umfaßt.

**Patentansprüche für den Vertragsstaat: AT**

1. Pflanzenwuchsregulierende Zusammensetzung, welche eine pflanzenwuchsregulierende Menge einer Verbindung der Formel I

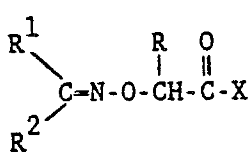

(I)

worin X für OH oder $OR^3$ steht, $R^3$ eine Alkyl- oder Alkoxyalkylgruppe mit 1 bis 10 Kohlenstoffatomen ist; R eine Alkylgruppe mit 2 bis 4 Kohlenstoffatomen darstellt; $R^1$ ein Wasserstoffatom oder eine Alkylgruppe mit 1 bis 6 Kohlenstoffatomen darstellt, welche wahlweise durch eine oder mehrere Halogenatome substituiert ist; und $R^2$ eine Alkyl- oder eine Alkenylgruppe mit bis zu 6 Kohlenstoffatomen, eine Phenylgruppe ist, welche Phenylgruppe wahlweise durch einen oder mehrere Substituenten, ausgewählt unter einem Halogenatom, einer Nitrogruppe, einer Alkyl-, Alkylthio- oder Alkoxygruppe mit 1 bis 4 Kohlenstoffatomen, welche jeweils wahlweise durch ein oder mehrere Halogenatome substituiert sind, oder durch eine Methylendioxygruppe substituiert ist, oder $R^1$ und $R^2$ zusammen mit dem Kohlenstoffatom, an welches sie begunden sind, eine cyklische Kohlenwasserstoffgruppe mit 3 bis 12 Kohlenstoffatomen bilden, gemeinsam mit mindestens einem Träger oder einem oberflächenaktiven Mittel umfaßt.

2. Zusammensetzung nach Anspruch 1, worin in Formel I R eine Ethyl- oder Isopropylgruppe ist; $R^1$ ein Wasserstoffatom oder eine Methyl-, Ethyl- oder Trifluoromethylgruppe darstellt; und $R^2$ eine Methylgruppe, eine 2-Methylpropenylgruppe, eine Phenylgruppe darstellt, welche durch eine oder zwei Gruppen, ausgewählt unter Chlor, Fluor, Nitro, Methyl oder Trifluoromethyl, oder durch Methylendioxy substituiert ist, oder eine Tetralinylgruppe ist.

3. Zusammensetzung nach Anspruch 2, worin $R^1$ ein Wasserstoffatom ist und $R^2$ 2,4-Dichlorphenyl oder 3-Nitrophenyl darstellt.

4. Zusammensetzung nach Anspruch 2, worin $R^1$ eine Methylgruppe ist und $R^2$ eine 3-Trifluormethylphenylenegruppe ist.

5. Verfahren zum Bekämpfen von Pflanzenwuchs an einem Ort, welches das Aufbringen einer bekämpfenden Menge einer wirksamen Verbindung nach Anspruch 1 oder einer Zusammensetzung nach Anspruch 1 auf den Ort oder die Pflanzen umaßt.

**Revendications pour les Etats contractants: BE CH DE FR GB IT LI LU NL SE**

1. Un composé de formule I

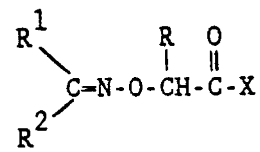

(I)

où X est H ou $OR^3$ où $R^3$ est un groupe alcoyle ou alcoxyalcoyle contenant de 1 à 10 atomes de carbone; R est un groupe alcoyle contenant de 2 à 4 atomes de carbone; $R^1$ est un atome d'hydrogène ou un groupe alcoyle contenant de 1 à 6 atomes de carbone éventuellement substitué par un ou plusieurs atomes d'halogènes; et $R^2$ est un groupe alcoyle ou alcényle contenant jusqu'à 6 atomes de carbone, un groupe phényle éventuellement substitué par un ou plusieurs substituants choisis parmi un atome d'halogène, un groupe nitro, un groupe alcoyle, alcoylthio ou alcoxy contenant de 1 à 4 atomes de carbone, chacun éventuellement substitué par un ou plusieurs atomes d'halogènes ou par un groupe méthylènedioxy, ou $R^1$ et $R^2$ en même temps que l'atome de carbone auquel ils sont attachés forment un groupe hydrocarbyle cyclique contenant de 3 à 12 atomes de carbone.

2. Un composé selon la revendication 1, dans lequel R est un groupe éthyle ou isopropyle; $R^1$ est un atome d'hydrogène ou un groupe méthyle, éthyle ou trifluorométhyle; et $R^2$ est un groupe méthyle, un groupe 2-méthylpropényle, un groupe phényle substitué par un ou deux groupes choisis parmi le chlore, le fluor et les groupes nitro, méthyle ou trifluorométhyle ou par un groupe méthylènedioxy, ou est un groupe tétralinyle.

3. Un composé selon la revendication 2, dans lequel $R^1$ est un atome d'hydrogène et $R^2$ est un groupe 2,4-dichlorophényle ou 3-nitrophényle.

4. Un composé selon la revendication 2, dans lequel $R^1$ est un groupe méthyle et $R^3$ est un groupe 3-trifluorométhylphényle.

5. Une composition pour régulation de la croissance de plantes comprenant une quantité convenable pour régulation de la croissance de plantes d'un composé actif selon la revendication 1, en même temps qu'au moins un véhicule ou un agent tensio-actif.

6. Un procédé de lutte contre la croissance de plantes en un lieu, qui comprend l'application à ce lieu ou aux plantes d'une quantité efficace d'un composé actif selon la revendication 1 ou d'une composition selon la revendication 5.

**Revendications pour l'Etat contractant: AT**

1. Une composition pour régulation de la croissance de plantes comprenant une quantité convenable pour régulation de la croissance de plantes d'un composé de formule I

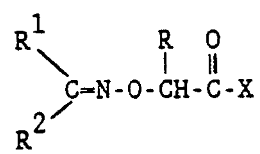

(I)

où X est H ou $OR^3$ où $R^3$ est un groupe alcoyle ou alcoxyalcoyle contenant de 1 à 10 atomes de carbone; R est un groupe alcoyle contenant de 2 à 4 atomes de carbone; $R^1$ est un atome d'hydrogène ou un groupe alcoyle contenant de 1 à 6 atomes de carbone éventuellement substitué par un ou plusieurs atomes d'halogènes; et $R^2$ est un groupe alcoyle ou alcényle contenant jusqu'à 6 atomes de carbone, un groupe phényle éventuellement substitué par un ou plusieurs substituants choisis parmi un atome d'halogène, un groupe nitro, un groupe alcoyle, alcoylthio ou alcoxy contenant de 1 à 4 atomes de carbone, chacun éventuellement substitué par un ou plusieurs atomes d'halogènes ou par un groupe méthylènedioxy, ou $R^1$ et $R^2$ en même temps que l'atome de carbone auquel ils sont attachés forment un groupe hydrocarbyle cyclique contenant de 3 à 12 atomes de carbone en même temps qu'au moins un véhicule ou un agent tensio-actif.

2. Une composition selon la revendication 1, dans laquelle, dans la formule I, R est un groupe éthyle ou isopropyle; $R^1$ est un atome d'hydrogène ou un groupe méthyle, éthyle ou trifluorométhyle; et $R^2$ est un groupe méthyle, un groupe 2-méthylpropényle, un groupe phényle substitué par un ou deux groupes choisis parmi le chlore, le fluor et les groupes nitro, méthyle ou trifluorométhyle ou par un groupe méthylènedioxy, ou est un groupe tétralinyle.

3. Une composition selon la revendication 2, dans laquelle $R^1$ est un atome d'hydrogène et $R^2$ est un groupe 2,4-dichlorophényle ou 3-nitrophényle.

4. Une composition selon la revendication 2, dans laquelle $R^1$ est un groupe méthyle et $R^3$ est un groupe 3-trifluorométhylphényle.

5. Un procédé de lutte contre la croissance de plantes en un lieu, qui comprend l'application à ce lieu ou aux plantes d'une quantité efficace d'un composé tel que défini dans la revendication 1 ou d'une composition selon la revendication 1.